# FASCICULE DE BREVET EUROPEEN

(11) **EP 3 716 903 B1**
(45) Date de publication et mention de la délivrance du brevet: **31.12.2025**
(21) Numéro de dépôt: 18819192.8
(22) Date de dépôt: 15.11.2018
(51) Int. Cl.: A61F 2/06, A61F 2/07, A61F 2/90

(54) **STRUCTURE OPTIMISÉE POUR UN IMPLANT EXPANSIBLE DU GENRE STENT OU ENDOPROTHESE**
OPTIMIERTE STRUKTUR FÜR EIN EXPANDIERBARES STENT- ODER ENDOPROTHESENIMPLANTAT
OPTIMISED STRUCTURE FOR AN EXPANDABLE IMPLANT OF THE STENT OR ENDOPROSTHESIS TYPE

(30) Priorité: 27.11.2017 FR 1761229
(43) Date de publication de la demande: 07.10.2020
(73) Titulaire: ID Nest Medical, 67000 Strasbourg (FR)
(72) Inventeur: WIECEK, William, 42160 Saint Cyprien (FR); CONTASSOT, David, 67230 Benfeld (FR)
(74) Mandataire: Merckling, Norbert
(86) Numéro de dépôt international: PCT/FR2018/052857
(87) Numéro de publication internationale: WO 2019/102110

(56) Documents cités:
- FR-A1- 2 858 208
- US-A1- 2010 241 218
- US-A1- 2017 304 093
- US-B1- 6 264 689

## Description

### Domaine technique

La présente invention se rapporte au domaine technique général des implants expansibles, appelés également stents ou endoprothèses. Ces implants sont destinés à être mis en place dans tout conduit de circulation d'un fluide corporel ou cavité d'un être vivant. Il peut s'agir d'un implant simple ou d'un implant connectable. Ce dernier est destiné à être lié mécaniquement une fois posé dans un conduit d'une bifurcation à un autre implant. L'implant est donc appelé soit implant mère, soit implant branche.

À titre d'exemple la mise en place d'implants, du genre stents ou stents connectables, s'effectue au niveau de la confluence de veines iliaques ou au niveau de la crosse aortique ou de toute confluence.

Les implants, par exemple connectés, sont par exemple posés dans une cavité d'un être vivant et plus particulièrement dans un réseau circulatoire notamment artériel et veineux, dans une région où le vaisseau correspondant présente des branches collatérales nécessitant le maintien de leur perfusion.

Dans un exemple d'application, le conduit de circulation du fluide, en l'occurrence le sang, correspond à l'aorte, dans laquelle est positionné un premier implant, un deuxième implant est déployé dans une artère branchée sur l'aorte telle que l'artère iliaque, l'artère rénale, l'artère mésentérique supérieure, le tronc cœliaque et les troncs artériels supra-aortiques, etc.

La présente invention peut donc s'appliquer au premier implant, au second implant ou aux deux.

Dans un autre exemple, le premier implant est destiné à être disposé dans la crosse aortique. Le deuxième implant est alors placé dans une des branches débouchant dans la crosse aortique, tels que l'artère carotide primitive gauche, l'artère sous-clavière gauche ou le tronc artériel brachiocéphalique.

L'invention concerne également les implants destinés à une application en urologie.

D'autres applications sont possibles dans le système veineux, notamment dans la veine cave inférieure au niveau de la bifurcation iliaque, au niveau de l'abouchement des veines rénales et de la veine cave supérieure ou au niveau de l'abouchement de ses branches collatérales.

Le ou les implants cités ci-dessus sont destinés à être implantés dans des conduits de circulation du sang notamment pour traiter des zones présentant des défauts ou des maladies tels que des anévrismes ou des dissections.

La mise en place d'implant conforme à l'invention est également utilisée dans des cas de maladie vasculaire tels que des lésions oblitérantes et des syndromes compressifs.

Les implantations en question se font généralement à l'aide de techniques endoluminales. Ces techniques mini-invasives, procurent moins de mortalité et de morbidité pour le patient ainsi qu'une diminution du temps opératoire.

### Etat de la technique

On connaît de nombreuses réalisations de stents ou d'implants et notamment divers procédés de fabrication. On connaît ainsi par exemple des stents réalisés à l'aide d'un tressage de fils métalliques. Ces derniers sont réalisés à l'aide d'un alliage appelé « NITINOL ».

Les implants connus, expansibles à l'aide de ballonnets ou auto-expansible, présentent souvent l'inconvénient de s'allonger lors de leur déploiement et de présenter ainsi une longueur supérieure à leur longueur nominale au repos. L'allongement maximal est atteint lorsque l'implant est comprimé et logé dans un lanceur. Lors de sa libération l'implant se déploie en diminuant de longueur. Cette diminution reste cependant insuffisante pour atteindre à nouveau la longueur nominale au repos. L'implant présente donc un diamètre plus faible que son diamètre nominal au repos, ce qui altère ses caractéristiques mécaniques, dont la résistance mécanique notamment. Un tel allongement a par exemple pour conséquence de diminuer la résistance de l'implant au bout d'un certain temps, pouvant conduire alors à un nouvel écrasement du conduit de circulation de fluide. Ceci peut correspondre par exemple dans une veine écrasée contre la colonne vertébrale. En outre, un implant branche doit conserver ses propriétés mécaniques et élastiques pour assurer la connexion optimale à un implant mère, tout en présentant une force radiale suffisante pour contrer l'écrasement. Lorsqu'un implant connu, de forme tubulaire présentant une extrémité de connexion pour s'ancrer par exemple sur un autre implant, est soumis à un écrasement même partiel, les organes de connexion prévus ont tendance à s'ouvrir. Il apparaît alors un jeu mécanique entre les implants connectés. Il peut donc en résulter une déconnexion entre l'implant branche et l'implant mère.

US2010/241218A1 divulgue un implant pourvu d'organes de connexion comprenant un bourrelet de maintien.

On connaît également, par l'intermédiaire du document US 6,264,289 B1 (D4), une technique de fabrication d'un stent à partir de fils tressés. Cette technique de tressage incorporant des fils torsadés et des fils tressés a pour but de réaliser un stent se comprimant plus facilement et dont le raccourcissement est maîtrisé lors de son déploiement. Ce document décrit que des fils torsadés selon une direction transversale sont combinés à au moins une intersection formée de fils tressés. Une telle configuration rend le procédé de fabrication complexe à mettre en œuvre d'une part et ne permet pas d'éviter un allongement lors de compressions radiales d'autre part. En outre, le stent décrit n'est pas un stent branche comportant des organes de connexion du genre comprenant une extrémité repliable ou un bourrelet.

On connaît également par l'intermédiaire du document US 2002/0147489 A1, une réalisation de stent, basée exclusivement sur l'utilisation du tressage de fils. Ce document, concernant plus particulièrement un procédé de fabrication d'un stent et l'outillage correspondant, décrit cependant des intersections de fils torsadés en association avec des intersections tressées. Ainsi, un stent représenté, est constitué d'un premier maillage dont les intersections sont formées de fils torsadés et d'un second maillage décalé par rapport au premier venant croiser ce dernier avec des intersections de fils tressés. Par construction, le stent ainsi obtenu ne présente pas de secteurs longitudinaux ne comportant que des fils torsadés et leur l'agencement ne permet pas d'éviter un allongement lors de compressions radiales. Le procédé de fabrication de tels stents est également complexe à mettre en œuvre.

Le document US 2017/304093 A1 (Dl) décrit un stent dont la structure tubulaire est réalisée avec des secteurs tressés. Ces derniers sont par exemple séparés par un secteur torsadé de manière à former un découplage entre les deux secteurs tressés. Ce dernier peuvent ainsi présenter un diamètre différent. Les stents décrits ne se rapportent à à de stenst branches purvus d'organes de connexion du genre comprenant une extrémité repliable ou un bourrelet.

### Exposé de l'invention

L'objet de la présente invention vise à proposer un nouvel implant dont le fonctionnement est optimal et fiable tout au long de son implantation.

Un objet de l'invention vise donc à proposer un nouvel implant dont l'allongement est substantiellement diminué ou nul par rapport à sa longueur nominale au repos, lorsque ledit implant est soumis à des forces radiales de compression, de compression locale transversale ou d'écrasement.

Un autre objet de la présente invention vise à fournir un nouvel implant mère ou implant branche présentant des propriétés mécaniques optimales tout en étant réalisé de manière simple et économique.

Un autre objet de la présente invention vise à fournir un nouvel ensemble d'implants connectés mécaniquement, comprenant un implant branche connecté sur un implant mère, dont la fiabilité de la liaison mécanique est conservée en cas de compression dudit implant branche.

Les objets assignés à l'invention sont atteints à l'aide d'un implant selon la revendication indépendante 1.

Selon un exemple de réalisation, le ou chaque secteur longitudinal torsadé comprend des torsades individuelles réparties sur l'intégralité du périmètre circulaire transversal dudit secteur longitudinal torsadé.

Selon un exemple de réalisation, au moins un secteur longitudinal torsadé comprend au niveau des croisements des fils métalliques, au moins deux torsades individuelles juxtaposées longitudinalement selon la direction de construction longitudinale parallèle à l'axe longitudinal et réparties sur l'intégralité du périmètre transversal dudit secteur longitudinal torsadé.

Selon un exemple de réalisation, la structure tubulaire comporte au moins deux secteurs longitudinaux torsadés distincts et espacés longitudinalement, localisés chacun entre deux secteurs longitudinaux tressés.

Selon un exemple de réalisation, des secteurs longitudinaux torsadés espacés longitudinalement dans la structure tubulaire sont constitués au niveau des croisements des fils métalliques d'un nombre différent de torsades individuelles juxtaposées.

Selon un exemple de réalisation, un premier secteur longitudinal torsadé et un second secteur longitudinal torsadé présentent au niveau de chaque croisement des fils métalliques, respectivement une construction avec une torsade individuelle et une construction avec trois torsades individuelles juxtaposées.

Selon un exemple de réalisation, chaque secteur longitudinal tressé présente pour chaque fil métallique un angle α, β compris entre 30° et 70°, préférentiellement compris entre 40° et 65° et très préférentiellement compris entre 45° et 60° par rapport à un axe longitudinal de la structure tubulaire.

A titre d'exemple, chaque secteur longitudinal tressé présente pour chaque fil métallique des angles β, α de +/- 60° par rapport à un axe longitudinal de la structure tubulaire.

Selon un autre exemple de réalisation, chaque secteur longitudinal tressé présente pour chaque fil métallique des angles β, α de +/- 45° par rapport à un axe longitudinal de la structure tubulaire.

Selon un exemple de réalisation, la structure tubulaire comprend un textile ou une membrane de couverture.

Selon un exemple de réalisation, l'implant est auto-expansible.

L'implant conforme à l'invention constitue un implant branche et en ce que la structure tubulaire comporte un secteur longitudinal tressé d'extrémité, pourvu d'organes de connexion destinés à un accrochage à un autre implant appelé implant mère.

Selon l'invention, les organes de connexion comprennent une extrémité périphérique repliable et un bourrelet de maintien.

Selon un exemple de réalisation, le secteur longitudinal tressé d'extrémité présente longitudinalement et successivement à partir de son extrémité libre, l'extrémité périphérique repliable, suivie du bourrelet de maintien, lequel est juxtaposé à un secteur longitudinal torsadé dans la structure tubulaire.

Les objets assignés à l'invention sont également atteints à l'aide d'un ensemble d'implants destinés à être connectés mécaniquement in situ dans un organe vivant, comprenant au moins un implant branche tel que présenté ci-dessus et un implant mère, lequel comporte des organes de connexion complémentaires destinés à coopérer avec les organes de connexion de l'implant branche.

Selon un exemple de réalisation, les organes de connexion complémentaires comportent une couronne élastique délimitant une ouverture intérieure dont une portion périphérique est destinée à se loger entre l'extrémité périphérique et le bourrelet une fois la connexion réalisée, la couronne élastique étant sollicitée élastiquement vers une position de repos à partir d'une position d'introduction présentant un diamètre supérieur à son diamètre dans la position de repos.

Un implant conforme à l'invention présente l'énorme avantage de procurer des propriétés mécaniques optimisées et adaptables aux diverses formes et usages dudit implant. Cet avantage concernant aussi bien un implant simple ou un implant mère, qu'un implant branche, est obtenu par une structure particulière permettant de limiter de façon substantielle, voire d'annuler l'allongement dudit implant en réponse à des efforts de compression. Ainsi, sur un plan mécanique, la structure tubulaire, même comprimée ou subissant un contrainte transversale, continue ou par cycles, ne se fragilise pas dans le temps et ne réduit pas ses performances. L'implant conforme à l'invention conserve ainsi son ouverture interne et par conséquent une ouverture non altérée du conduit dans lequel il est implanté.

L'implant conforme à l'invention comporte ainsi la structure tubulaire, laquelle présente un allongement substantiellement diminué ou nul par rapport à sa longueur nominale au repos, lorsque ladite structure tubulaire est soumise à des forces radiales de compression, des forces de compression locale transversale ou des forces d'écrasement.

Un autre avantage de l'invention réside dans une fabrication automatisée de l'implant, se traduisant notamment, par des coûts de fabrication avantageux.

Un autre avantage de l'implant conforme à l'invention est obtenu car le changement de configuration de la structure tubulaire intégrant des secteurs exclusivement tressés et des secteurs exclusivement torsadés, n'altère pas sa faculté à être comprimé dans un cathéter en vue d'une pose avec une technique endovasculaire. La structure de l'implant conforme à l'invention n'altère ni ses fonctionnalités, ni son utilisation de façon mini-invasive en chirurgie endoscopique.

Un autre avantage de l'implant conforme à l'invention réside dans sa compatibilité avec une membrane ou un textile pour constituer une endoprothèse recouverte. Ainsi, on évite une mise en forme spécifique souvent complexe d'une membrane ou couverture, pour s'adapter à l'allongement de l'implant ou stent. En effet, avec les implants recouverts connus, présentant un allongement, la couverture ou la membrane doit être en mesure de recouvrir l'implant déployé et dont la longueur est supérieure à sa longueur nominale au repos. Ceci est techniquement souvent compliqué à atteindre de manière fiable.

Un autre avantage d'un implant ou stent sans allongement conforme à l'invention, par exemple un stent branche, réside dans le fait que le chirurgien n'a pas à gérer la vitesse de libération dudit stent pour éviter un allongement trop important. En effet, les frictions du stent sur les parois du conduit et/ou l'ancrage à une extrémité, s'opposent à un retour à sa longueur nominale au repos dans un état déployé. Ce phénomène est accentué avec une vitesse de libération ou de largage importante.

Un autre avantage de l'invention réside dans une précision accrue du positionnement de l'implant dans un conduit. En effet, un allongement mal maîtrisé de l'implant déployé et posé peut par exemple conduire à un positionnement non optimal vis-à-vis de la zone à traiter ou à un positionnement avec une extrémité qui débouche dans l'autre conduit d'une bifurcation et favorisant ainsi l'obstruction dudit autre conduit.

Un autre avantage est obtenu avec un implant branche conforme à l'invention comportant des organes de connexion lui permettant de se raccorder à un autre implant. En effet, de par sa construction, l'implant branche permet de réaliser une connexion dont la fiabilité n'est pas altérée même en cas de compression ou d'effort transversal ou radial exercé sur la structure tubulaire. L'implant branche conforme à l'invention permet d'éviter l'apparition d'un jeu mécanique lequel serait préjudiciable à la fiabilité de sa connexion avec l'autre implant. Ainsi, quels que soient les compressions subies, l'implant branche présente un allongement nul ou minimal et en tous cas remarquablement maîtrisé.

### Brève description des figures

D'autres caractéristiques avantages de la présente invention apparaîtront plus clairement à la lecture de la description qui va suivre, faite en référence aux dessins annexés, donnés à titre d'exemples non limitatifs, dans lesquels:
- la figure 1, est une illustration partielle d'un exemple de réalisation d'un implant conforme à l'invention,
- la figure 2, est une illustration partielle d'un autre exemple de réalisation d'un implant conforme à l'invention,
- la figure 3 est un agrandissement d'une partie de l'implant conforme à l'invention représenté à la figure 1,
- la figure 4 est un agrandissement d'une partie de l'implant conforme à l'invention représenté à la figure 2,
- les figures 5, 6, 7 et 8, illustrent, avec des représentations schématiques des exemples de réalisation additionnels d'implants conformes à l'invention,
- la figure 9, illustre un exemple de réalisation d'un implant conforme à l'invention,
- les figures 10 et 11 illustrent le comportement en réponse à une compression de l'implant conforme à l'invention, et
- la figure 12 illustre un exemple de réalisation d'un ensemble d'implants connectés conforme à l'invention.

### Exposé détaillé de l'invention

Les éléments structurellement et fonctionnellement identiques et présents sur plusieurs figures distinctes, sont affectés d'une même référence numérique ou alphanumérique.

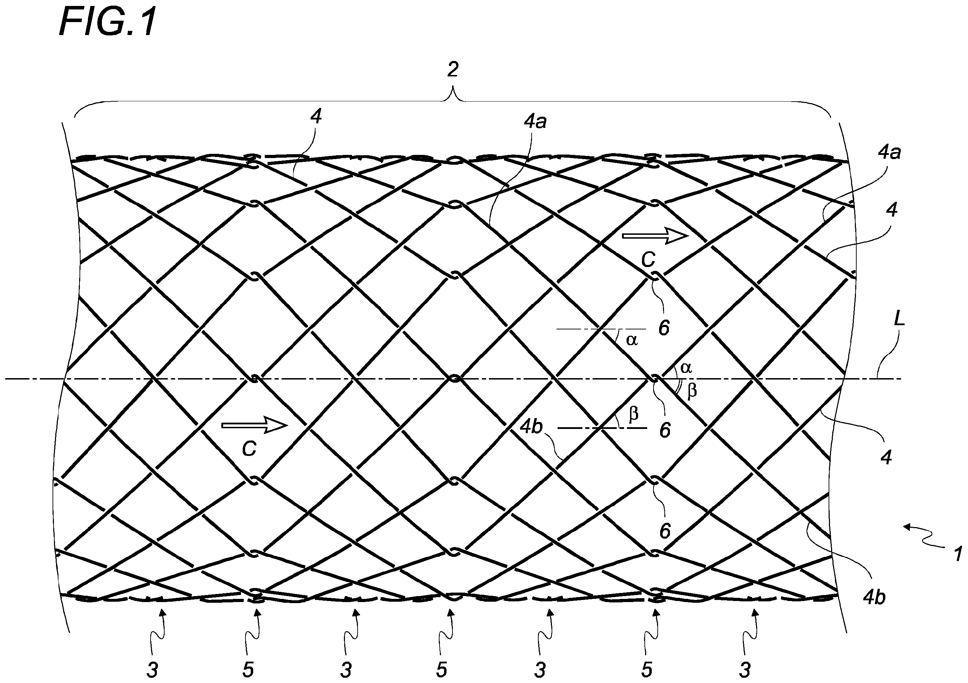

Un exemple de réalisation d'un implant 1 est par exemple représenté à la figure 1 dans un état déployé. L'implant 1 conforme à l'invention comprend une structure tubulaire 2 s'étendant selon un axe longitudinal L.

La structure tubulaire 2 comprend des secteurs longitudinaux tressés 3 de fils métalliques 4. Ces derniers peuvent également être appelés brins ou filaments. Les fils métalliques 4 présentent avantageusement un diamètre inférieur à 0,4 mm.

La structure ou plus précisément la partie de la structure tubulaire 2 représentée à la figure 1 comporte quatre secteurs longitudinaux tressés 3. Ces derniers sont obtenus de préférence à l'aide d'au moins deux fils métalliques 4.

Selon un autre mode de fabrication, il est envisageable de réaliser les secteurs tressés 3 par l'intermédiaire d'un unique fil métallique 4.

La structure tubulaire 2 présente des extrémités longitudinales ouvertes pour assurer le passage d'un fluide. La structure tubulaire 2 comprend de préférence à chacune de ses extrémités longitudinales un secteur longitudinal tressé 3.

La structure tubulaire 2 comprend également trois secteurs longitudinaux torsadés 5, lesquels sont réalisés avec les fils métalliques 4 pour former en continuité ladite structure tubulaire 2.

Dans l'exemple de réalisation illustré à la figure 1, chaque secteur longitudinal torsadé 5 comprend une couronne de torsades individuelles 6. Ainsi à chaque localisation d'une torsade individuelle 6, les fils métalliques 4 issus d'un secteur longitudinal tressé 3 se croisent et sont repliés mutuellement pour ensuite s'étendre chacun selon une direction d'extension de l'autre fil métallique 4 qu'ils ont croisé et constituer ainsi, dans le prolongement longitudinal de la couronne de torsades individuelles 6, un autre secteur longitudinal tressé 3. Les torsades individuelles 6 présentent ainsi une direction de construction parallèle à l'axe longitudinal L.

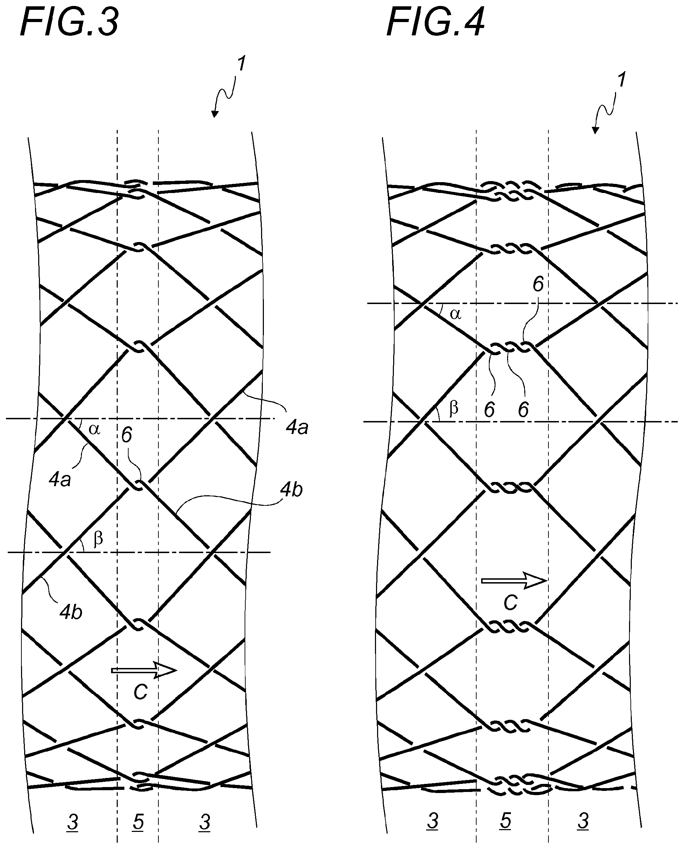

À des fins d'illustration, nous avons identifié dans la structure tubulaire 2, notamment aux figures 1 et 3, un premier fil métallique 4a participant à un secteur longitudinal tressé 3 ainsi qu'un second fil métallique 4b participant au même secteur longitudinal tressé 3.

Le fil métallique 4a présente avantageusement un angle α de - 45° avec un axe longitudinal de la structure tubulaire 2 et le fil métallique 4b présente un angle β de + 45° avec un axe longitudinal. Ces axes longitudinaux sont parallèles à l'axe longitudinal L de la structure tubulaire 2.

Les fils métalliques 4a et 4b se croisent pour constituer une torsade individuelle 6 en étant repliés l'un sur l'autre pour repartir dans la direction de l'autre fil métallique respectif 4b et 4a après la réalisation de ladite torsade individuelle 6. Le fil métallique 4a présente alors avantageusement un angle α de + 45° avec un axe longitudinal de la structure tubulaire 2 et le fil métallique 4b présente alors un angle β de - 45° avec un axe longitudinal.

Les torsades individuelles 6 d'une même couronne sont donc avantageusement alignées et réparties sur un tracé sensiblement circulaire de la structure tubulaire 2.

La figure 3 est un agrandissement d'une partie de la figure 1, illustrant plus en détail la couronne de torsades individuelles 6 réalisant le secteur longitudinal torsadé 5.

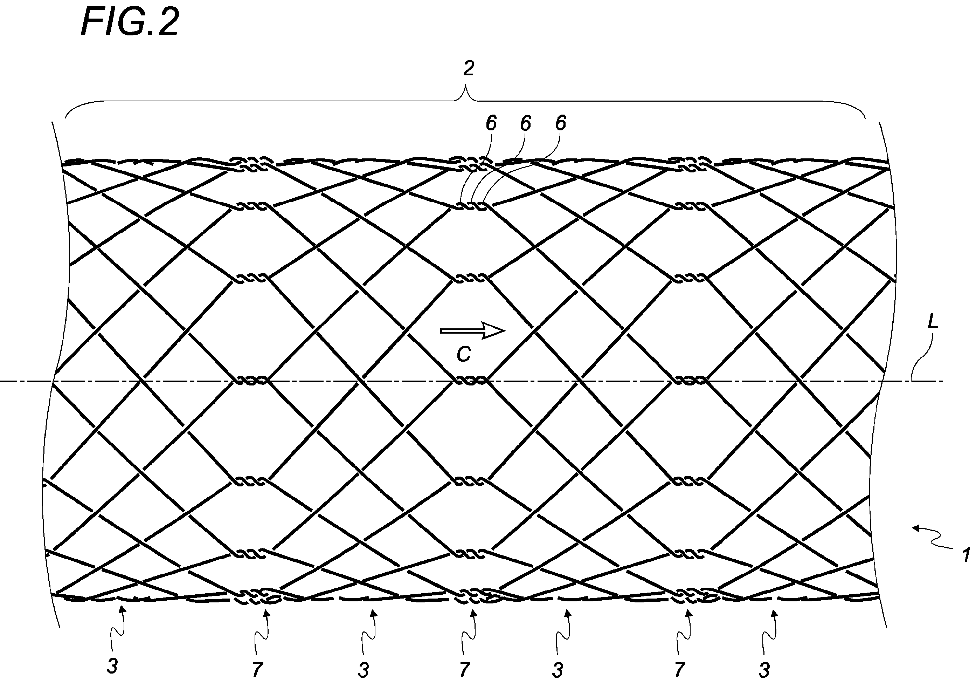

La figure 2 illustre un autre exemple de réalisation d'un implant dans lequel la structure tubulaire 2 comprend trois secteurs longitudinaux torsadés 7. Chaque secteur longitudinal torsadé 7 comprend plusieurs couronnes de torsades individuelles 6 juxtaposées longitudinalement. En l'occurrence chaque secteur torsadé longitudinal 7 comporte trois couronnes juxtaposées.

Le secteur torsadé longitudinal 7 présente donc une succession d'interconnexions de deux fils métalliques 4 réparties sur un tracé ou un périmètre circulaire de la structure tubulaires 2. Chaque interconnexion comprend par conséquent une juxtaposition de trois torsades individuelles 6 construites selon une direction longitudinale C.

La figure 3 illustre avec une vue agrandie d'une partie de la structure tubulaire 2 de la figure 1, le secteur longitudinal torsadé 5 comportant une torsade individuelle 6 à chaque interconnexion des fils métalliques 4 pour constituer une couronne de torsades.

La figure 4 illustre avec une vue agrandie d'une partie de la structure tubulaire 2 de la figure 2, le secteur longitudinal torsadé 7 comportant trois torsades individuelles 6 juxtaposées à chaque interconnexion des fils métalliques 4 pour constituer trois couronnes de torsades juxtaposées.

Dans les exemples de réalisation illustrés aux figures 1 et 2, les secteurs longitudinaux tressés 3 sont réalisés avec des fils métalliques 4 présentant par rapport à une direction longitudinale un angle α et β de +/- 45°.

Selon un autre exemple de réalisation de l'implant conforme à l'invention, non représenté aux figures, ces angles α et β sont de +/- 60°. On pourra se reporter par exemple aux figures 3 et 4 sur lesquelles sont reportés les angles α et β.

La structure tubulaire 2 d'un implant 1 comporte par exemple plusieurs secteurs longitudinaux torsadés 5 ou 7 distincts, localisés chacun entre deux secteurs longitudinaux tressés 3. Chacun des secteurs longitudinaux torsadés 5,7 peut ainsi présenter une dimension longitudinale plus ou moins grande et être espacés d'un autre secteur longitudinal torsadé 5,7 en fonction des propriétés mécaniques recherchées, à savoir sa capacité à résister à un écrasement radial ou localisé (non périphérique) et sa flexibilité.

Un secteur torsadé longitudinal 7 présente par exemple une dimension longitudinale plus importante dans la mesure où il intègre trois couronnes de torsades individuelles 6 successives, comparativement à un secteur longitudinal torsadé 5 ne comportant qu'une couronne de torsades individuelles 6.

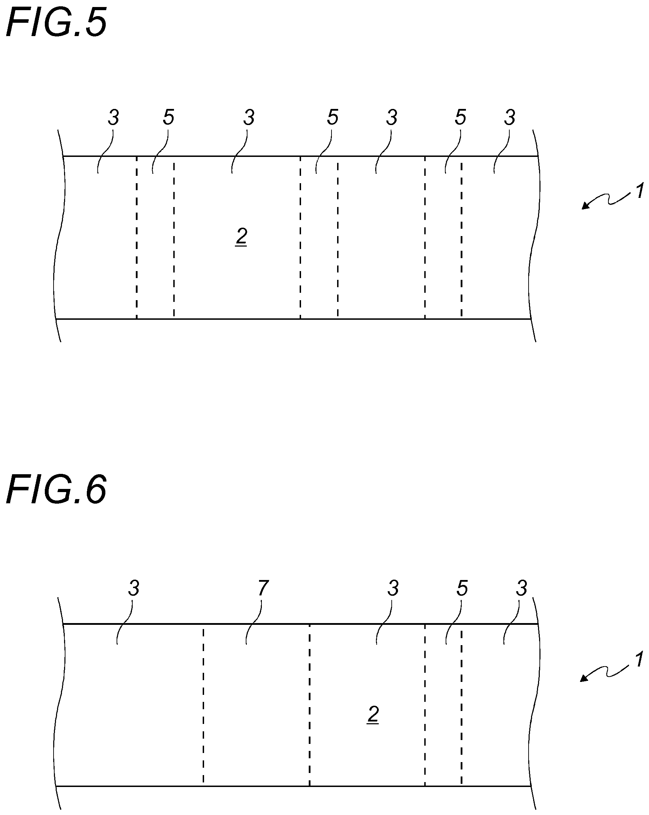

Un tel exemple est par exemple illustré schématiquement à la figure 6.

Selon un autre exemple de réalisation illustrée à la figure 5 la structure tubulaire 2 comprend trois secteurs longitudinaux torsadés 5 présentant un espacement longitudinal mutuel différent. Il est ainsi possible d'obtenir des propriétés mécaniques différentes le long de l'implant 1 ou stent.

Dans chaque secteur torsadé 5 ou 7, les torsades individuelles 6, juxtaposées ou non, présentent une direction de construction longitudinale C, parallèle à l'axe longitudinal L de la structure tubulaire 2.

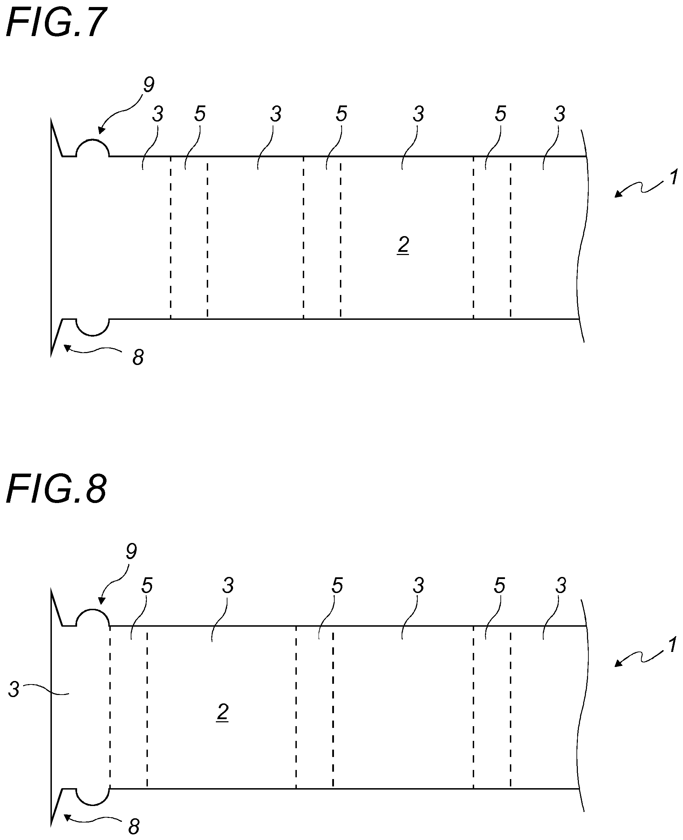

La figure 7 illustre un exemple de réalisation d'un implant 1, lequel comprend une extrémité périphérique repliable 8 ainsi qu'un bourrelet de maintien 9 pour former des organes de connexion. Ces derniers sont destinés à l'accrochage dans une fenêtre d'un autre implant. L'implant illustré schématiquement à la figure 7 est donc appelé implant branche.

La figure 8 illustre un autre exemple de réalisation d'un implant 1 conforme à l'invention dans lequel le secteur longitudinal tressé 3 d'extrémité présente longitudinalement et successivement à partir de son extrémité libre, l'extrémité périphérique repliable 8, puis le bourrelet de maintien 9. Ce dernier est avantageusement juxtaposé à un secteur longitudinal torsadé 5. Le secteur longitudinal torsadé 5 s'étend de préférence dans le prolongement du bourrelet de maintien 9.

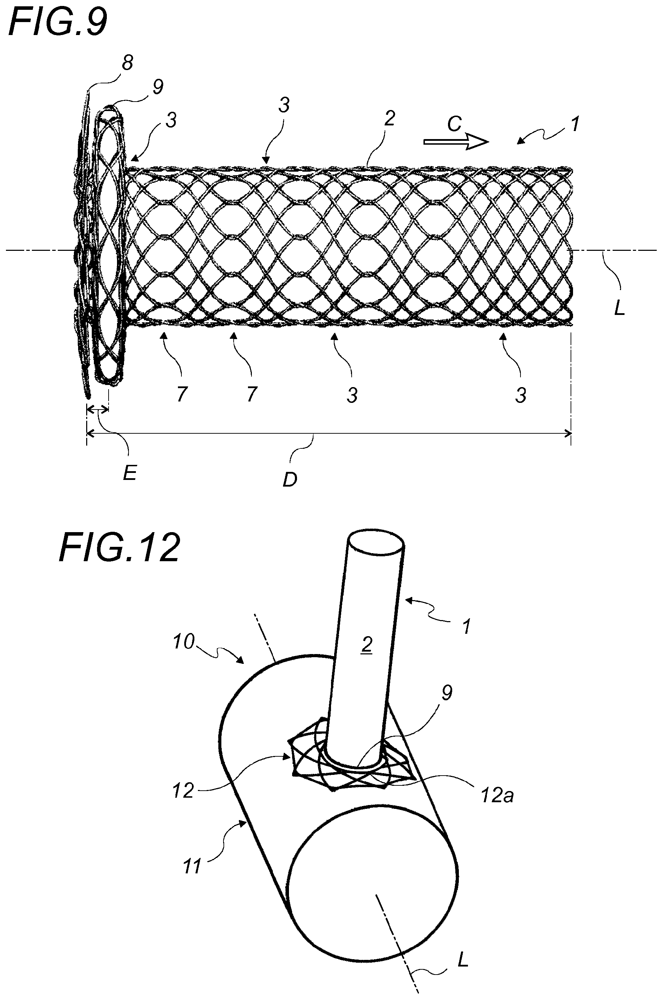

La figure 9, illustre un exemple de réalisation d'un implant 1 branche dans son état déployé. L'implant 1 branche, une fois largué et déployé, présente une longueur globale D ainsi qu'un écartement E entre l'extrémité périphérique repliable 8 et le bourrelet 9.

L'écartement entre le bourrelet 9 et le secteur torsadé 5 ou 7 situé dans son prolongement est de préférence quasi nul ou minimisé. Cela contribue à éviter une apparition d'un jeu mécanique pouvant altérer la connexion entre deux implants.

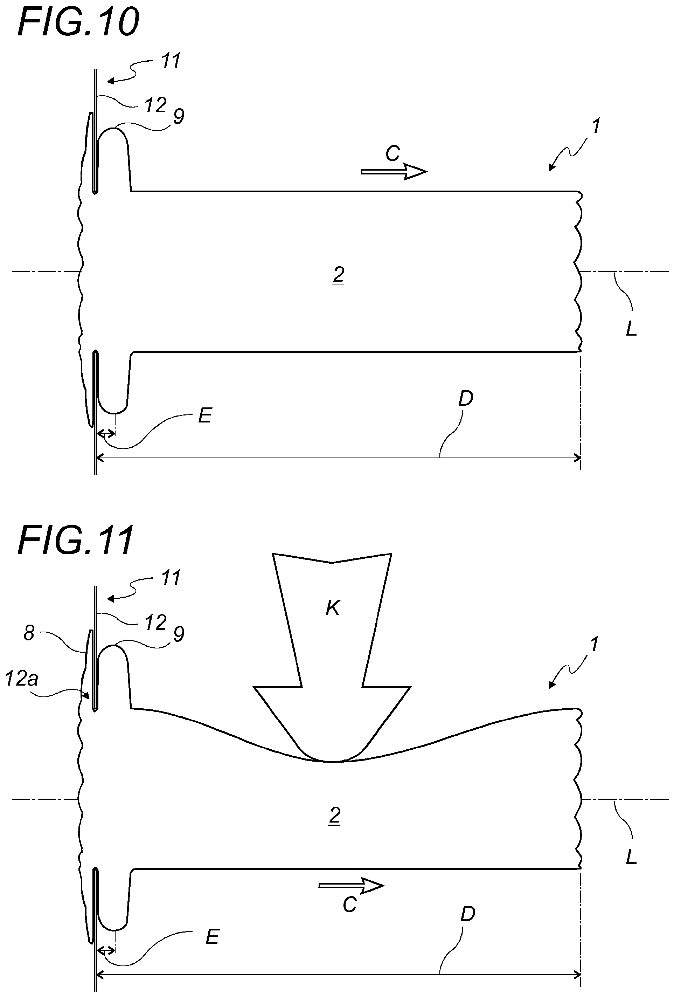

Une contrainte mécanique, radiale ou non, sur la structure tubulaire 2, n'aura ainsi qu'un impact limité voir nul sur la forme des organes de connexion dans leur configuration déployée. On pourra par exemple se reporter aux figures 10 et 11.

Les figures 10 et 11 illustrent le comportement en réponse à une compression K radiale sur la structure tubulaire 2 de l'implant 1 branche. Un autre implant 11, appelé implant 11 mère comporte avantageusement une ouverture délimité par une couronne élastique 12 dont une portion périphérique 12a est destinée à se loger entre l'extrémité périphérique repliable 8 et le bourrelet 9 une fois le connexion réalisée.

La couronne élastique 12 est alors sollicitée élastiquement vers une position de repos à partir d'une position d'introduction présentant un diamètre supérieur à son diamètre dans la position de repos.

La figure 11 illustre remarquablement que l'implant 1 branche, soumis à une compression radiale K n'augmente ni l'écartement E entre l'extrémité périphérique repliable 8 et le bourrelet 9, ni sa longueur globale D. la fiabilité de la connexion entre les deux implants 1 et 11 n'est donc pas altérée.

La figure 12 illustre selon une vue en perspective un exemple de réalisation schématique d'un ensemble 10 d'implants connectés conforme à l'invention. Ainsi, l'ensemble 10 d'implants comprend au moins un implant 1 branche et un autre implant 11 mère, lequel comporte les organes de connexion complémentaires destinés à coopérer avec les organes de connexion de l'implant 1 branche.

L'implant 1 branche conforme à l'invention, présente avantageusement une élongation inférieure à 5% lors d'une compression radiale, alors qu'un implant 1 branche de l'art antérieur de mêmes dimensions, présente une élongation supérieure à 22 % lors d'une compression radiale identique.

La présente invention trouve également son application dans le domaine des endoprothèses recouvertes par un textile ou une membrane.

De manière évidente, l'invention ne se limite pas au mode de réalisation ou de mise en œuvre préférentiel décrit précédemment et représenté sur les différentes figures, l'homme du métier pouvant y apporter de nombreuses modifications et imaginer d'autres variantes sans sortir du cadre de l'invention défini par les revendications.

## Revendications

1. Implant (1) expansible pour l'implantation dans un conduit ou dans une cavité d'un être vivant, comprenant une structure tubulaire (2) s'étendant selon un axe longitudinal (L), ladite structure tubulaire (2) comportant au moins deux secteurs longitudinaux tressés (3) formé avec des fils métalliques (4) tressés et des extrémités longitudinales ouvertes, **caractérisé en ce que** la structure tubulaire (2) comporte un desdits secteurs longitudinaux tressés (3) à chaque extrémité longitudinale de la structure tubulaire (2) et au moins un secteur longitudinal torsadé (5,7) formé avec des fils métalliques torsadés (5,7) pour former en continuité avec lesdits secteurs longitudinaux tressés (3) ladite structure tubulaire (2), lesdits secteurs longitudinaux tressés (3) et ledit au moins un secteur longitudinal torsadé (5,7) étant de révolution et ledit au moins un secteur longitudinal torsadé (5,7) comportant des torsades (6) présentant chacune une direction de construction longitudinale (C) parallèle à l'axe longitudinal (L) et **en ce qu'**il constitue un implant (1) branche dont un desdits secteurs tressés (3) d'extrémité est pourvu d'organes de connexion lesquels comprennent une extrémité périphérique repliable (8) et un bourrelet de maintien (9) et lesquels sont destinés à un accrochage à un autre implant (11) appelé implant mère, la structure tubulaire (2) présentant un allongement longitudinal substantiellement diminué ou nul par rapport à sa longueur nominale au repos, lorsque ladite structure tubulaire (2) est soumise à des forces radiales de compression, de compression locale transversale ou d'écrasement.

2. Implant (1) selon la revendication 1, **caractérisée en ce que** chaque secteur longitudinal torsadé (5,7) comprend des torsades individuelles (6) réparties sur l'intégralité du périmètre circulaire transversal dudit secteur longitudinal torsadé (5,7).

3. Implant (1) selon la revendication 2, **caractérisé en ce qu'**au moins un secteur longitudinal torsadé (5,7) comprend au niveau des croisements des fils métalliques (4), au moins deux torsades individuelles (6) juxtaposées longitudinalement selon la direction de construction longitudinale (C) parallèle à l'axe longitudinal (L) et réparties sur l'intégralité du périmètre transversal dudit secteur longitudinal torsadé (5).

4. Implant (1) selon l'une quelconque des revendications 1 à 3, **caractérisé en ce que** la structure tubulaire (2) comporte au moins deux secteurs longitudinaux torsadés (5,7) distincts et espacés longitudinalement, localisés chacun entre deux secteurs longitudinaux tressés (3).

5. Implant (1) selon la revendication 4, **caractérisé en ce que** des secteurs longitudinaux torsadés (5,7) espacés longitudinalement dans la structure tubulaire (2) sont constitués au niveau des croisements des fils métalliques (4) d'un nombre différent de torsades individuelles (6) juxtaposées.

6. Implant (1) selon la revendication 5, **caractérisé en ce qu'**un premier secteur longitudinal torsadé (5) et un second secteur longitudinal torsadé (7) présentent au niveau de chaque croisement des fils métalliques (4), respectivement une construction avec une torsade individuelle (6) et une construction avec trois torsades individuelles (6) juxtaposées.

7. Implant (1) selon l'une quelconque des revendications 1 à 6, **caractérisé en ce que** chaque secteur longitudinal tressé (3) présente pour chaque fil métallique (4) un angle α, β compris entre 30° et 70°, préférentiellement compris entre 40° et 65° et très préférentiellement compris entre 45° et 60° par rapport à un axe longitudinal (L) de la structure tubulaire (2).

8. Implant (1) selon l'une quelconque des revendications 1 à 7, caractérisé en que la structure tubulaire (2) comprend un textile ou une membrane de couverture.

9. Implant (1) selon l'une quelconque des revendications 1 à 8, **caractérisé en ce qu'**il est auto-expansible.

10. Implant (1) selon l'une quelconque des revendications 1 à 9, **caractérisé en ce que** le secteur longitudinal tressé (3) d'extrémité pourvu d'organes de connexion, présente longitudinalement et successivement à partir de son extrémité libre, l'extrémité périphérique repliable (8), suivie du bourrelet de maintien (9), lequel est juxtaposé à un secteur longitudinal torsadé (5,7) dans la structure tubulaire (2).

11. Ensemble d'implants (10) destinés à être connectés mécaniquement in situ dans un organe vivant, comprenant au moins un implant (1) branche conforme à l'une quelconque des revendications 1 à 10 et un implant (11) mère, lequel comporte des organes de connexion complémentaires destinés à coopérer avec les organes de connexion de l'implant (1) branche.

12. Ensemble d'implants (10) selon la revendication 11, **caractérisé en ce que** les organes de connexion complémentaires comportent une couronne élastique (12) délimitant une ouverture intérieure dont une portion périphérique (12a) est destinée à se loger entre l'extrémité périphérique (8) et le bourrelet (9) une fois la connexion réalisée, la couronne élastique (12) sollicitée élastiquement vers une position de repos à partir d'une position d'introduction présentant un diamètre supérieur à son diamètre dans la position de repos.

## Patentansprüche

1. Ausdehnbares Implantat (1) zur Implantation in einen Kanal oder eine Vertiefung eines Lebewesens, mit einer röhrenförmigen Struktur (2), die sich an einer Längsachse (L) entlang erstreckt, wobei die röhrenförmigen Struktur (2) mindestens zwei geflochtene longitudinale Abschnitte (3) umfasst, die aus geflochtenen Metalldrähten (4) gebildet sind und offene longitudinale Enden aufweisen, **dadurch gekennzeichnet, dass** die röhrenförmige Struktur (2) einen der geflochtenen longitudinalen Abschnitte (3) an jedem longitudinalen Ende der röhrenförmigen Struktur (2) und mindestens einen verdrillten longitudinalen Abschnitt (5, 7) aufweist, der mit verdrillten Metalldrähten (5, 7) gebildet ist, um in Kontinuität mit den geflochtenen longitudinalen Abschnitten (3) die röhrenförmige Struktur (2) zu bilden, wobei die geflochtenen longitudinalen Abschnitte (3) und der mindestens eine verdrillte longitudinale Abschnitt (5, 7) rotationssymmetrisch sind und der genannte mindestens eine verdrillte longitudinale Abschnitt (5, 7) Verdrillungen (6) aufweist, die jeweils eine longitudinale Konstruktionsrichtung (C) parallel zur Längsachse (L) aufweisen, und dass es ein Zweigimplantat (1) bildet, dessen einer der geflochtenen Abschnitte (3) am Ende mit Verbindungselementen versehen ist, die ein peripheres, umlegbares Ende (8) und einen Rückhaltewulst (9) umfassen, und die dazu bestimmt sind, an einem anderen Implantat (11), dem sogenannten Mutterimplantat, befestigt zu werden, wobei die röhrenförmige Struktur (2) eine im Vergleich zu ihrer Nennlänge im Ruhezustand erheblich verringerte oder keine Längsdehnung aufweist, wenn die röhrenförmige Struktur (2) radialen Druckkräften, lokalen Querdruckkräften oder Quetschkräften ausgesetzt ist.

2. Implantat (1) nach Anspruch 1, **dadurch gekennzeichnet, dass** jeder verdrillte longitudinale Abschnitt (5, 7) einzelne Verdrillungen (6) umfasst, die über den gesamten kreisförmigen Umfang in Qerrichtung des verdrillten longitudinalen Abschnitts (5, 7) verteilt sind.

3. Implantat (1) nach Anspruch 2, **dadurch gekennzeichnet, dass** mindestens ein verdrillter longitudinaler Abschnitt (5, 7) an den Kreuzungspunkten der Metalldrähte (4) mindestens zwei einzelne Verdrillungen (6) umfasst, die in Längsrichtung entsprechend der Konstruktion in Längsrichtung (C) parallel zur Längsachse (L) angeordnet sind und sich über den gesamten Querumfang des verdrillten longitudinalen Abschnitts (5) erstrecken.

4. Implantat (1) nach einem der Ansprüche 1 bis 3, **dadurch gekennzeichnet, dass** die röhrenförmige Struktur (2) mindestens zwei unterschiedliche und in Längsrichtung voneinander beabstandete verdrillte longitudinale Abschnitte (5, 7) aufweist, die jeweils zwischen zwei geflochtenen longitudinalen Abschnitten (3) angeordnet sind.

5. Implantat (1) nach Anspruch 4, **dadurch gekennzeichnet**, **dadurch gekennzeichnet, dass** verdrillte longitudinale Abschnitte (5, 7), die in Längsrichtung in der röhrenförmigen Struktur (2) beabstandet sind, an den Kreuzungspunkten der Metalldrähte (4) aus einer unterschiedlichen Anzahl nebeneinander angeordneter einzelner Verdrillungen (6) bestehen.

6. Implantat (1) nach Anspruch 5, **dadurch gekennzeichnet**, **dadurch gekennzeichnet, dass** ein erster verdrillter longitudinaler Abschnitt (5) und ein zweiter verdrillter longitudinaler Abschnitt (7) an jeder Kreuzung der Metalldrähte (4) jeweils eine Konstruktion mit einer einzelnen Verdrillung (6) und eine Konstruktion mit drei nebeneinander angeordneten einzelnen Verdrillungen (6) aufweisen.

7. Implantat (1) nach einem der Ansprüche 1 bis 6, **dadurch gekennzeichnet**, **dadurch gekennzeichnet, dass** jeder geflochtene longitudinale Abschnitt (3) für jeden Metalldraht (4) einen Winkel α, β zwischen 30° und 70°, vorzugsweise zwischen 40° und 65° und besonders bevorzugt zwischen 45° und 60° in Bezug auf eine Längsachse (L) der röhrenförmigen Struktur (2) aufweist.

8. Implantat (1) nach einem der Ansprüche 1 bis 7, **dadurch gekennzeichnet, dass** die röhrenförmige Struktur (2) ein Textil oder eine Abdeckmembran umfasst.

9. Implantat (1) nach einem der Ansprüche 1 bis 8, **dadurch gekennzeichnet, dass** es selbstexpandierend ist.

10. Implantat (1) nach einem der Ansprüche 1 bis 9, **dadurch gekennzeichnet, dass** der mit Verbindungselementen versehene geflochtene longitudinale Abschnitt (3) am Ende in Längsrichtung und aufeinanderfolgend von seinem freien Ende aus das perihere, umlegbare Ende (8), gefolgt von dem Rückhaltewulst (9) aufweist, der neben einem verdrillten longitudinalen Abschnitt (5, 7) in der röhrenförmigen Struktur (2) angeordnet ist.

11. Implantatset (10), das dazu bestimmt ist, mechanisch in situ in einem lebenden Organ verbunden zu werden, das mindestens ein Zweigimplantat (1) nach einem der Ansprüche 1 bis 10 und ein Mutterimplantat (11) umfasst, das komplementäre Verbindungselemente umfasst, die dazu bestimmt sind, mit den Verbindungselementen des Zweigimplantats (1) zusammenzuwirken.

12. Implantatset (10) nach Anspruch 11, **dadurch gekennzeichnet, dass** die komplementären Verbindungselemente einen elastischen Kranz (12) umfassen, der eine innere Öffnung begrenzt, deren Umfangsbereich (12a) dazu bestimmt ist, zwischen dem peripheren Ende (8) und dem Wulst (9) aufgenommen zu werden, sobald die Verbindung herestellt wurde, wobei der elastische Kranz (12) aus einer Einführposition, deren Durchmesser größer ist als sein Durchmesser in der Ruhestellung, elastisch in eine Ruhestellung vorgespannt ist.

## Claims

1. Expandable implant (1) for implantation in a duct or in a cavity of a living being, comprising a tubular structure (2) extending along a longitudinal axis (L), said tubular structure (2) comprising at least two braided longitudinal sectors (3) formed with braided metal wires (4) and open longitudinal ends, **characterised in that** the tubular structure (2) comprises one of said braided longitudinal sectors (3) at each longitudinal end of the tubular structure (2) and at least one twisted longitudinal sector (5, 7) formed with twisted metal wires (5, 7) to form, in continuity with said braided longitudinal sectors (3), said tubular structure (2), said braided longitudinal sectors (3) and said at least one twisted longitudinal sector (5, 7) being rotational and said at least one twisted longitudinal sector (5, 7) comprising twists (6) each having a longitudinal construction direction (C) parallel to the longitudinal axis (L) and **in that** it constitutes a branch implant (1), one of said end braided sectors (3) of which is provided with connecting members which comprise a foldable peripheral end (8) and a retaining flange (9) and which are intended for attachment to another implant (11) called a mother implant, the tubular structure (2) having a longitudinal elongation that is substantially reduced or zero with respect to its nominal length at rest, when said tubular structure (2) is subjected to radial forces of compression, local transverse compression or crushing.

2. Implant (1) according to claim 1, **characterised in that** each twisted longitudinal sector (5, 7) comprises individual twists (6) distributed over the entire transverse circular perimeter of said twisted longitudinal sector (5, 7).

3. Implant (1) according to claim 2, **characterised in that** at least one twisted longitudinal sector (5, 7) comprises, at the junctions of the metal wires (4), at least two individual twists (6) longitudinally juxtaposed in the longitudinal construction direction (C) parallel to the longitudinal axis (L) and distributed over the entire transverse perimeter of said twisted longitudinal sector (5).

4. Implant (1) according to any of claims 1 to 3, **characterised in that** the tubular structure (2) includes at least two separate twisted longitudinal sectors (5, 7) spaced apart longitudinally, each located between two braided longitudinal sectors (3).

5. Implant (1) according to claim 4, **characterised in that** twisted longitudinal sectors (5, 7) spaced apart longitudinally in the tubular structure (2) are constituted, at the junctions of the metal wires (4), by a different number of individual juxtaposed twists (6).

6. Implant (1) according to claim 5, **characterised in that** a first twisted longitudinal sector (5) and a second twisted longitudinal sector (7) have, at each junction/interconnection of the metal wires (4), respectively a construction with an individual twist (6) and a construction with three individual juxtaposed twists (6).

7. Implant (1) according to any of claims 1 to 6, **characterised in that** each braided longitudinal sector (3) has, for each metal wire (4), an angle α, β that lies in the range 30° to 70°, preferentially in the range 40° to 65° and more preferentially in the range 45° to 60° relative to a longitudinal axis (L) of the tubular structure (2).

8. Implant (1) according to any of claims 1 to 7, **characterised in that** the tubular structure (2) comprises a covering membrane or textile.

9. Implant (1) according to any of claims 1 to 8, **characterised in that** it is self-expandable.

10. Implant (1) according to any one of claims 1 to 9, **characterised in that** the end braided longitudinal sector (3) provided with connecting members has, longitudinally and successively from the free end thereof, the folding peripheral end (8), followed by the retaining flange (9), which is juxtaposed with a twisted longitudinal sector (5, 7) in the tubular structure (2).

11. Set of implants (10) intended to be mechanically connected in situ in a living being, comprising at least one branch implant (1) according to any of claims 1 to 10 and a parent implant (11), which includes complementary connecting members intended to engage with the connecting members of the branch implant (1).

12. Set of implants (10) according to claim 11, **characterised in that** the complementary connecting members include a resilient ring (12) delimiting an inner opening, a peripheral portion (12a) whereof is intended to be housed between the peripheral end (8) and the flange (9) once the connection has been made, the resilient ring (12) being elastically stressed towards a resting position from an insertion position having a diameter that exceeds the diameter thereof in the resting position.
